(19) Europäisches Patentamt
European Patent Office
Office européen des brevets

(11) **EP 3 272 311 B1**

(12) **EUROPEAN PATENT SPECIFICATION**

(45) Date of publication and mention
of the grant of the patent:
**03.04.2024 Bulletin 2024/14**

(21) Application number: **17275104.2**

(22) Date of filing: **12.07.2017**

(51) International Patent Classification (IPC):
***A61F 2/01*** *(2006.01)*

(52) Cooperative Patent Classification (CPC):
**A61F 2/0105;** A61F 2210/0004; A61F 2210/0014;
A61F 2210/0076; A61F 2220/0016;
A61F 2230/0067; A61F 2240/001; A61F 2250/0015;
A61F 2250/0029

(54) **IMPLANTABLE MEDICAL DEVICE AND METHOD OF MANUFACTURE**

IMPLANTIERBARE MEDIZINISCHE VORRICHTUNG UND VERFAHREN ZU HERSTELLUNG

DISPOSITIFS MÉDICAUX ET PROCÉDÉS DE FABRICATION

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **21.07.2016 GB 201612653**

(43) Date of publication of application:
**24.01.2018 Bulletin 2018/04**

(73) Proprietor: **Cook Medical Technologies LLC
Bloomington, IN 47404 (US)**

(72) Inventors:
• **Johnsen, Jeppe
5871 Froerup (DK)**
• **Dela, Christian
4621 Gadstrup (DK)**
• **Mølgaard-Nielsen, Arne
2100 Copenhagen (DK)**
• **Hansen, Palle
4632 Bjaeverskov (DK)**
• **Klausen, Kasper
4623 Lille Skensved (DK)**
• **Løvdal, Alexandra
2920 Charlottenlund (DK)**

(74) Representative: **Williams Powell
44 Prospect Place
Bromley, Kent BR2 9HN (GB)**

(56) References cited:
**WO-A1-2015/192019 GB-A- 2 531 587**

## Description

Technical Field

The present invention relates to implantable medical devices such as vascular filters and methods of forming them and/or struts for medical devices.

Background of the Invention

Several vena cava filters with different advantages and disadvantages are known in the art. Conventionally, a vena cava filter is placed through access in the jugular or femoral vein using the Seldinger technique. Once having placed a predilator and an introducer sheath via a guide wire and verified the position with a cavogram, the filter is implanted, generally by pushing an introducer system on which the filter is carried through the introducer sheath. When at the intended location, the filter is released.

Inferior vena cava (IVC) filters are commonly conical and comprise a series of filter struts or legs which are designed to press against the wall of the vena cava. This facilitates the fixation in the vena cava and prevents the IVC filter migrating cranially along the vessel wall.

Examples of known devices are described in US-8,092,484, US-2012/0143238, US-2003/0153972, EP-2,208,479, US-2003/0135265, US-2008/0281393, US-2012/0130470, US-2012/0221040, US-2013/0006294, US-2013/0204349, US-6,517,559, US-7,575,584, US-8,162,970, WO-2013/106746, WO-2009/041664 and US-2010/0016940.

GB 2531587 discloses an implantable medical device exhibiting diminishing radial force.

WO 2015/192019 discloses a biodegradable wire with central filament.

Summary of the present disclosure

The present invention seeks to provide an improved implantable medical device and method of forming implantable medical devices and/or struts therefor.

According to an aspect of the present invention, there is provided an implantable medical device including a plurality of struts arranged in a generally conical form; each strut having a first end, a second end, and an operative length between the first and second ends; each strut being able to flex along the operative length thereof and including:

a strut skeleton having a first flexural modulus; and a reinforcement element extending along the operative length and attached to the strut skeleton, wherein the reinforcement element and the strut skeleton together have a second flexural modulus greater than the first flexural modulus, wherein at least one of the reinforcement element and the strut skeleton varies in at least one of amount and composition continuously along the operative length such that the second flexural modulus varies in a continuous manner along the operative length; wherein the reinforcement element is biodegradable thereby changing the flexural modulus of the strut from the second flexural modulus to the first flexural modulus as the reinforcement element biodegrades.

The terms reinforcing element and reinforcement element are used interchangeably.

In some embodiments of the disclosure a filter is provided with fixation filter struts in which the flexural modulus of the fixation filter struts reduces over time, and therefore the radial force with which they press against the vessel wall reduces over time. This means that the filter exhibits a higher radial force immediately after implantation when it is important for the filter to secure itself in the vessel. Initially, embodiments can also provide the fixation filter struts with a flexural modulus that varies along an operative length of the struts, providing control over the provision of the radial outward force. However, after a period of time, for example within which the fixation filter struts have been encapsulated by tissue, the flexural modulus of the fixation filter struts is no longer necessary, and in embodiments of the invention it is accordingly reduced. Furthermore, some embodiments of the disclosure are able to provide fixation filter struts having a profile which provides a continuous or smooth curve of radial outward force along the struts. This can avoid step-changes, and preferably also sharp changes in gradient, in the force on the filter strut and avoid potential trauma or increased ingrowth that a step-change might otherwise produce. It can also mean that the filter can conveniently be used with different sized vessels as the radial outward force on the vessel is easy to predict irrespective of where along the fixation filter strut it contacts the vessel.

Preferably, the second flexural modulus varies in a smooth manner along the operative length, that is to say without step-changes in the value of the flexural modulus or sharp changes in the gradient of the flexural modulus with respect to distance.

The strut skeleton and/or the reinforcement element for each strut can be provided with different amounts, cross-sections, diameters, materials and/or ratios of materials at different points along the operative length of the strut. In some embodiments, the diameter of the reinforcement element and/or the strut skeleton varies along the operative length of each strut.

In some embodiments, the device includes a hub element to which the first end of the strut is attached, the struts extending at least partially radially from the hub element towards the second end of the strut.

In some embodiments, each reinforcement element extends continuously along the operative length of the strut.

Preferably, a radial outward force provided by each strut is substantially uniform or continuously or smoothly varying along the operative length.

In some embodiments, each strut skeleton and its respective reinforcement element are integrally formed.

In some embodiments the device struts form a material capture basket.

In some embodiments, the device is a vascular filter or occluder.

In some embodiments, the second end of each strut is a free end.

In preferred embodiments, the strut skeleton has a first end, a second end, and an operative length between the first and second ends. The first flexural modulus can be uniform or vary in a continuous or smooth manner along the operative length of the strut and/or strut skeleton.

Preferably, each strut has an area moment of inertia which is one of uniform or varying in a continuous or smooth manner along the operative length of the strut; and/or each strut skeleton has an area moment of inertia which is one of uniform or varying in a continuous or smooth manner along an operative length of the strut and/or strut skeleton.

The area moment of inertia of the strut skeleton is referred to herein as a first area moment of inertia and the area moment of inertia of the reinforced strut is referred to herein as the second area moment of inertia.

In some embodiments, the diameter or cross-section of each strut skeleton is uniform along the operative length of the strut and/or strut skeleton.

Preferably, a radial outward force provided by each strut skeleton after degradation of the reinforcement element is substantially uniform or continuously or smoothly varying along the operative length of the strut skeleton.

In preferred embodiments, the continuousness of the flexural modulus means that there are no apices, that is to say no step-changes or jumps, in the flexural modulus along the operative length of the struts, either immediately upon implantation or after the degradation time of the reinforcing element. This can avoid points of high-stress both immediately after implantation and after the degradation time. Points of high-stress in a filter strut could potentially be traumatic and/or increase ingrowth of the filter strut.

In some embodiments, the operative length of each strut and/or each strut skeleton is a flexure length. The term flexure length is used herein to refer to a length along which a strut or strut skeleton is configured to flex for example to provide a radially expansive force.

In some embodiments the operative length of a strut is the entire length of the respective strut and/or the operative length of a strut skeleton is the entire length of the respective strut skeleton.

In some embodiments, the flexural modulus and/or area moment of inertia can be greatest at a first end of the operative length of the strut or strut skeleton, which is towards the hub element, and lowest at a second end of the operative length of the strut or strut skeleton, which is away from the hub element. This can be the case initially, in which case the flexural modulus and area moment of inertia would be the second flexural modulus and second area moment of inertia; after the reinforcing element has degraded, in which case the flexural modulus and area moment of inertia would be the first flexural modulus and first area moment of inertia; and/or throughout degradation of the reinforcing element.

Throughout degradation of each reinforcing element, the flexural modulus and/or the area moment of inertia can be one of uniform or continuously or smoothly varying along the operative length of the strut, that is to say at all points in time.

In some embodiments, along the operative length of each strut a gradient of the second flexural modulus and/or second area moment of inertia with respect to distance along the strut is always greater than or equal to zero, or always less than or equal to zero. In some embodiments, along an operative length of each strut and/or strut skeleton a gradient of the first flexural modulus and/or first area moment of inertia with respect to distance along the strut skeleton is always greater than or equal to zero or always less than or equal to zero. In some embodiments, along the operative length of each strut a gradient of the flexural modulus and/or area moment of inertia with respect to distance along the strut is always greater than or equal to zero, or always less than or equal to zero, throughout degradation of the reinforcing element. In other words, in some embodiments, peaks or troughs in the value of the flexural modulus and/or area moment of inertia are avoided.

In some embodiments, for each strut, the reinforcement element extends along the entire length or entire operative length of the strut skeleton.

Preferably, along the operative length of each strut the flexural modulus of the strut is one of uniform or continuously or smoothly varying throughout degradation of the reinforcement element, which can mean that points of high-stress in the strut can be avoided at all points during the life of the medical device. In some embodiments, each reinforcing element is configured so that there is a uniform reduction in flexural modulus along the operative length, or along the entire length, of the strut, during degradation of the reinforcing element.

Preferably, along the operative length of each strut the area moment of inertia of the strut is one of uniform or continuously or smoothly varying throughout degradation of the reinforcement element. In some embodiments, each reinforcing element is configured so that there is a uniform reduction in area moment of inertia along the operative length, or along the entire length, of the strut, during degradation of the reinforcing element.

Preferably, each reinforcement element reinforces only the strut skeleton to which it is attached. That is to say that the reinforcement element does not couple multiple strut skeletons together.

In some embodiments, each strut skeleton and its respective reinforcement element together are a composite material.

Some embodiments include a hub element to which the first end of each strut and/or each strut skeleton is attached.

In some embodiments, each strut skeleton is formed of non-biodegradable material.

In some embodiments, each strut skeleton is formed of or comprises spring steel.

In some embodiments, a perpendicular cross-section of each strut and/or strut skeleton is elongate.

In some embodiments, a perpendicular cross-section of each strut and/or strut skeleton is oval-shaped, elliptical or rectangular.

In some embodiments, each strut skeleton is tubular and the reinforcement element is disposed at least partially within the tubular strut skeleton. In some embodiments, the entirety of the reinforcing element can be disposed within the tubular strut skeleton. In some embodiments, the reinforcing element can fill the entirety of the interior of the tubular strut skeleton. In some embodiments, the interior of the tubular strut skeleton can be free of the reinforcing element.

Each strut skeleton can be positioned off-centre within the reinforcement element.

According to an aspect of the disclosure, there is provided an implantable medical device including a strut, the strut including a strut skeleton embedded within a biodegradable reinforcing element, wherein the strut skeleton is positioned off-centre within the reinforcing element.

In some embodiments, the strut skeleton can be positioned radially inwardly of a centre of the strut. In some embodiments, the strut skeleton can be positioned radially outwardly of a centre of the strut.

The reinforcement element can be a coating over the strut skeleton.

The strut skeleton can include a plurality of wires or strands, which may in some embodiments be coupled together by the reinforcement element. The wires or strands are, in some embodiments, radially adjacent and coupled radially. In other words, the wires or strands are preferably not longitudinally coupled which might provide longitudinal breaks in the strut skeleton. The reinforcement element can coat each wire or strand of the strut skeleton, to provide a partial or full coating of each wire or strand of the strut skeleton. to an aspect of the disclosure, there is provided an implantable medical device including a strut, the strut including a plurality of wires or strands coupled together with biodegradable material.

The biodegradable material can be said to provide a reinforcing element.

The plurality of strands or wires may provide a strut skeleton.

The biodegradable material can be a partial or full coating over each wire or strand.

In some embodiments, the plurality of wires or strands can be in the form of a tubular strut skeleton and the biodegradable material can be disposed at least partially within the strut skeleton. In some embodiments, the entirety of the biodegradable material can be disposed within the tubular strut skeleton. In some embodiments, the biodegradable material can fill the entirety of the interior of the tubular strut skeleton.

In other embodiments, the plurality of wires or strands can form a substantially solid structure.

The strut is preferably a one-piece strut.

The strut skeleton may have a first flexural modulus which is uniform or continuously or smoothly varying along an operative length of the strut and/or skeleton.

The strut may have a second flexural modulus which is uniform or continuously or smoothly varying along an operative length of the strut.

In some embodiments, the medical device can be a vascular filter.

In some embodiments, the strut is substantially solid. In other embodiments, the strut can be hollow.

The plurality of wires or strands can be braided or coiled together.

The plurality of wires or strands can be twisted together in a helical configuration, for example with a pitch of from 0° to 90°. In some embodiments, 0° < pitch < 90°.

In some embodiments, the plurality of wires or strands is in the form of a tubular structure.

In some embodiments, the strut includes at least six wires or strands.

The medical device may include a plurality of said struts.

According to an aspect of the invention, there is provided a method of forming an implantable medical device, including:

forming a plurality of struts, each strut having a first end, a second end, an operative length between the first and second ends and a strut skeleton having a first flexural modulus;

forming a reinforcement element extending along the operative length of each strut, the reinforcement element and the strut skeleton together having a second flexural modulus greater than the first flexural modulus, wherein at least one of the reinforcement element and the strut skeleton varies in at least one of amount and composition continuously along the operative length such that the second flexural modulus varies in a continuous manner along the operative length;

arranging the struts into a generally conical frame; wherein the reinforcement element is biodegradable thereby changing the flexural modulus of the strut from the second flexural modulus to the first flexural modulus as the reinforcement element biodegrades.

In some embodiments, each reinforcing element is formed so as to be attached to the respective strut skeleton.

In some embodiments, each reinforcement element can be attached to the respective strut skeleton after the strut skeleton is formed. In other embodiments, they are formed integrally.

Preferably, the second flexural modulus varies in a smooth manner along the operative length of each strut.

Preferably, the second flexural modulus is varying in a smooth manner along the operative length of each strut.

Preferably, forming a reinforcement element extending along the operative length of each strut provides the filter strut with a second area moment of inertia which is one of uniform or varying in continuous or smooth manner along the operative length of the strut.

Preferably, forming each strut includes: selecting a desired first force or deflection profile for the strut; and determining a reinforcement profile for the strut skeleton in accordance with the first force or deflection profile and forming the reinforcement element extending along the operative length of each strut in accordance with the reinforcement profile thereby to provide the strut with the second flexural modulus and the first force or deflection profile and optionally the second area moment of inertia.

Preferably, the first force or deflection profile is substantially uniform or continuously or smoothly varying along the operative length of the strut.

Preferably, the reinforcement profile of the reinforcement element for each strut skeleton provides the strut with a flexural modulus and optionally area moment of inertia which is one of uniform or varying continuously or smoothly along the operative length of the strut throughout degradation of the reinforcing element.

The method preferably includes the step of selecting at least one of a desired intermediate force and/or deflection profile for during degradation of the reinforcing element such that the strut exhibits said intermediate force and/or deflection profile during degradation of the reinforcement element. The intermediate force and/or deflection profile is preferably uniform or continuously or smoothly varying along the operative length of the strut.

Forming each strut can include calculating the second flexural modulus, for example from the first force or deflection profile, and determining the reinforcement profile at least in part from the calculated second flexural modulus.

Forming each strut can include calculating the second area moment of inertia, for example from the first force or deflection profile, and determining the reinforcement profile at least in part from the calculated second area moment of inertia.

The step of determining the reinforcement profile can include determining a difference between the second flexural modulus and the first flexural modulus, and determining the reinforcement profile such that the addition of the reinforcing element in accordance with the reinforcement profile increases the flexural modulus of the strut by the said difference.

The step of determining the reinforcement profile can include determining a difference between the second area moment of inertia and the first area moment of inertia, and determining the reinforcement profile such that the addition of the reinforcing element in accordance with the reinforcement profile increases the area moment of inertia of the strut by the said difference.

Preferably, the method includes the step of selecting a desired second force or deflection profile for each strut skeleton; and

forming or selecting the strut skeleton in accordance with the second force or deflection profile such that the strut skeleton has the first flexural modulus and the second force or deflection profile and optionally first area moment of inertia.

The second force or deflection profile is preferably uniform or continuously or smoothly varying along the operative length of the strut and/or strut skeleton.

Forming each strut can include calculating a time dependent flexural modulus, and/or time dependent area moment of inertia, for the strut for during degradation of the reinforcing element, for example from the first, second and/or intermediate force or deflection profile, and determining the reinforcement profile at least in part from the calculated time dependent flexural modulus and/or time dependent area moment of inertia.

In some embodiments, the time dependent flexural modulus and/or time dependent area moment of inertia is/are one of uniform or continuously or smoothly varying along the operative length of the strut throughout degradation of the reinforcement element.

In some embodiments, the step of determining the reinforcement profile includes determining a perpendicular cross-section for the strut for example along the full operative length of the strut, for example using the second flexural modulus, second area moment of inertia, time dependent flexural modulus and/or time dependent area moment of inertia. For example, determining the reinforcement profile can be performed such that the strut has a predetermined first cross-section and/or a predetermined cross-section throughout degradation of the reinforcing element.

Some embodiments include the step of selecting or determining a perpendicular cross-section for the strut skeleton for example along the full operative length of the strut and/or strut skeleton, for example using the first flexural modulus and/or first area moment of inertia, and forming or selecting the strut skeleton with that perpendicular cross-section.

The strut or struts referred to above in connection with a filter are preferably fixation struts. In embodiments, such fixation struts contribute to the filtering function of the filter.

In some embodiments described herein the flexural modulus can be varied by changing the cross-sectional dimension and combination of composite elements.

The higher the flexural modulus, the stiffer the material.

In some embodiments, the reinforcing element or biodegradable material is a co-material, for example a biodegradable wire, or can be a polymer for example acting as a resin.

Some embodiments of the disclosure are constructed of a composite material, the matrix of the composite being a biodegradable polymer with which it is possible to attain a desired reduction of the bending moment and thereby the radial force through degradation of the material, and the fiber material of the composite being non-degradable material or different materials, which determine the resulting flexural modulus. Using this composite, the radial force acting on the vena cava can be designed within a frame of properties including radial force at implantation in vena cava, radial force during indwelling, period for reduction of radial force and final implant radial force. When the device according to this embodiment is encapsulated by tissue, radial force for fixation can be reduced to a minimum.

A reinforcement layer of a biodegradable material can secure the requirement of a short term spring force.

By constructing the non-degradable element of some embodiments of the disclosure to be very flexible, long term flexibility can be met.

In some embodiments, the elastic modulus (E-modulus) is constant for each material. The E-modulus of the composite can be varied by the combination of composite materials and determined computationally. By varying the composite elastic modulus or flexural modulus the degree of deflection or force at any given cross section of the filter strut can be achieved.

Brief Description of the Drawings

Embodiments of the invention are described below, by way of example only, with reference to the accompanying drawings, in which:

Figure 1 are schematic representations of an example of an existing conically shaped IVC filter in situ in a vessel of a patient;
Figure 2 is a side elevational view of a filter according to an embodiment of the invention;
Figure 3 is a longitudinal cross-sectional view through a part of a fixation strut of the filter of Figure 2;
Figure 4 is a transverse cross-sectional view through a fixation filter strut of the filter of Figure 2;
Figure 5a is a graph of flexural modulus against length along a fixation strut of the filter of Figure 2;
Figure 5b is a graph of the area moment of inertia against time for an embodiment of fixation strut for the filter of Figure 2;
Figure 5c is a graph of flexural modulus against time for the embodiment of fixation strut of Figure 5b;
Figure 5d shows a graph of degree of deflection against time for the embodiment of fixation strut of Figure 5b;
Figure 6 is a schematic side view of a filter according to an embodiment of the invention;
Figure 7 is a longitudinal cross-sectional view through a part of a fixation filter strut of the filter of Figure 6;
Figure 8 is a transverse cross-sectional view of a strut for a filter according to another embodiment of the invention; and
Figures 9 to 13 show other configurations of filter struts according to other embodiments of the invention.

Description of the Preferred Embodiments

Reference is made to "Celect Interior Vena Cava Wall Strut Perforation Begets Additional Strut Perforation", Journal of Vascular and Interventional Radiology October 2015 Volume 26 Issue 10 Pages 1510-1518.

Described below are preferred embodiments of an implantable medical device constructed according to the teachings herein. It is to be understood that the drawings are not to scale and are intended to be merely illustrative of the features and elements of the device and its components.

The preferred embodiment is a vena cava (IVC) filter of generally conical form. It is to be understood that the teachings herein are applicable to other types of medical device including filters of double conical form, occluders having a conical or double conical support frame and so on.

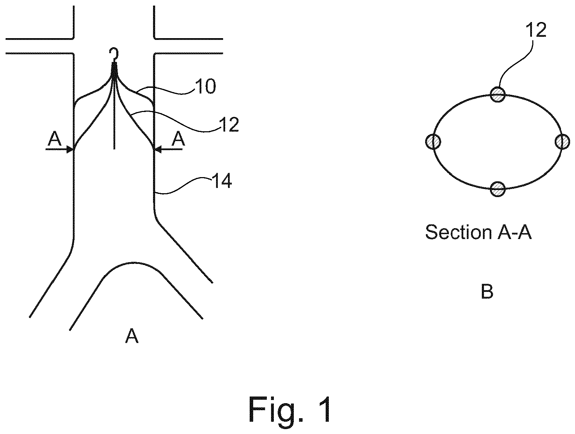

Figure 1a shows an example of a known IVC filter 10 in position in a vena cava 14. Figure 1b shows a transverse section through A-A in Figure 1a, in other words showing a transverse cross-section of at the open or wide end of the filter.

While a relatively high radial force can be advantageous on initial implantation in order to secure the filter in position and prevent or reduce the likelihood of migration of the filter in the vessel, this radial force can become undesirable after struts 12 have been encapsulated by tissue.

In the described embodiments, a filter is provided with fixation filter struts in which the flexural modulus of the struts reduces over time, causing the radial force with which they press against the vessel wall also to reduce over time. This causes the filter to exhibit a higher radial force immediately after implantation, when it is important for the filter to secure itself in the vessel. Initially, described embodiments can also provide the fixation filter struts with a flexural modulus that varies along an operative length of the struts, providing control over the provision of the radial outward force. After a period of time, during which the fixation filter struts have been encapsulated by tissue, the higher flexural modulus of the fixation filter struts is no longer necessary, and is accordingly reduced. Furthermore, described embodiments are able to provide fixation filter struts having a profile which provides a continuous curve of radial outward force along the length of the struts. Having a continuous curve of radial force avoids any step changes in the force applied by the filter struts, which could otherwise result in force apices along the strut which might cause trauma or localised damage to the vessel wall. A force curve which is non-stepped, that is continuous or continuously varying can also mean that the filter can conveniently be used with different sized vessels as the radial outward force on the vessel is easy to predict irrespective of where along the fixation filter strut the filter contacts the vessel. Preferably, the force curve is smooth, meaning no sharp changes in the gradient of the curve. In some embodiments, the radial outward force can be uniform along the struts, allowing for the same outward force to be applied, irrespective of the size of the vessel.

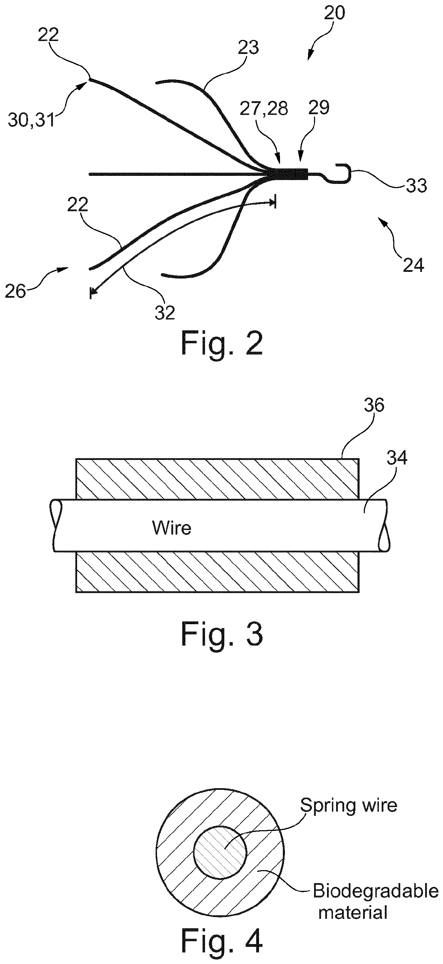

A filter 20 according to an embodiment of the invention is shown in Figure 2. Figure 2 shows the filter 20 in a radially expanded non-biased configuration, that is not radially compressed as it would be when deployed in a vessel having a diameter less than the maximum diameter of the filter structure.

The filter 20 includes a first, closed, end 24 and a second, wide, end 26, and a plurality of fixation filter struts 22 extending between the two ends 24, 26. The filter is able to be radially compressed such that the struts 22 are brought together to a diameter similar or the same as that of the first end 24. The struts 22 are also able to expand radially outwardly, as shown, into a filtering configuration. The filter 20 is designed to adopt the radially expanded filtering configuration when in a deployed condition, subject to any compressive force applied to the wide end 26 of the assembly by the walls of a vessel in which the filter 20 is deployed.

The fixation filter struts 22 may prevent cranial migration and may also prevent caudal migration.

The filter 20 also includes, in this embodiment, a plurality of stabilisation filter struts 23. The fixation filter struts 22 are used for securing the filter in a vessel and for filtering, whereas the stabilisation filter struts 23 are for stabilising the filter 20 against tilting. There is typically a plurality of stabilisation filter struts 23 disposed radially. There will typically be four to six stabilisation struts but the number can vary and in some cases can be omitted altogether.

The stabilisation struts 23 may in some embodiments be provided with the strength to withstand pressure in a filter occluded by thrombus to provide a filtration function in addition to that of the primary filter struts 22 and for this purpose may be interdigitated with the principal struts 22.

The filter struts 22, 23 have a springiness which is configured to bias the filter 20 towards the radially expanded filtering configuration. For this purpose, the struts 22, 23 include a springy material, such as spring steel or a shape memory alloy such as nickel titanium alloy (Nitinol being a commonly used material), or any of the other alloys known in the art.

Each of the fixation filter struts 22 includes a first end 28 and a second end 30. Each of the stabilisation filter struts 23 also includes a first end and a second end. In this embodiment, the first ends 28 of the fixation filter struts 22 and the first ends of the stabilisation filter struts 23 are coupled together at a filter hub 29, providing a closed end of the filter 20. The filter hub 29 is advantageously coupled to a coupling hook 33 at its first end 24. The hook 33 can conveniently be formed as a single component with the hub 29, for instance from a common length of tubing. The ends of the struts 22 and 23 can be fitted into the bore of the tube and fixed therein, by a friction fit, by welding, with a bonding agent or in any other suitable manner.

The second ends 30 of the fixation filter struts 22 are free ends and are located at the second end 26 of the filter 20. The second ends of the stabilisation filter struts 23 are also free ends but, as can be seen in Figure 2, do not extend all the way to the second end of the filter 20. As a result, the wide end of the stabilisation element formed by the struts 23 is longitudinally spaced from the wide end formed by the ends of the struts 22 so as to create two spaced vessel contact points. These spaced contact points act to stabilise the filter 20 in a vessel and prevent excessive tilting thereof.

In the deployed or radially expanded filtering configuration, each of the filter struts 22, 23 is configured to extend at least partially radially and distally from the filter hub 29.

In some embodiments there may be barbs or fixation hooks 35 at the second ends 30 of the fixation filter struts 22. Similar barbs or hooks may be provided at the free ends of the struts 23.

Each of the fixation filter struts 22 has an operative length 32, which in this embodiment is from the filter hub 29 to the second end 30 of the fixation filter strut 22. The operative length 32 can be described as a flexure length of the fixation filter strut, that is a length of the strut which is designed to flex in order to provide a radially expansive force. The flexure length can be a free length of the fixation filter strut 22 which is beyond a coupling region to the hub 29.

In this embodiment the operative length 32 is the entire length of each fixation filter strut 22.

As can be seen in Figure 2, in the radially expanded configuration, the filter struts 22 form a frame to the filter 20 which has a conical or tulip shape. This frame constitutes a material capture basket, in the case of a filter this being the filter basket. The fixation filter struts 22 allow blood to pass between them but catch larger particles such as emboli and other debris in the bloodstream. However, it is not excluded that there may be provided an additional filtering element such as a membrane attached to the filter struts, which in practice can provide finer filtration. An impermeable or substantially impermeable covering can cause the element 20 to act as an occluder or vascular plug.

The hook 33, shown in Figure 2, enables the filter 20 to be retrieved and removed from the vessel, as is known in the art, and can also assist in the initial deployment and repositioning of the filter 20. The hub 29 holds the components of the filter together so the filter can be retrieved by the hook 33.

In the preferred embodiment, each of the fixation filter struts 22 is made up of a strut skeleton 34 and a reinforcing element 36. This can be seen more clearly in Figures 3 and 4. An individual fixation filter strut 22 is discussed below but all of the fixation filter struts 22 are advantageously configured in the manner described. In some embodiments, the stabilisation filter struts 23 can also be configured in the manner described below. However, this is not generally necessary because the stabilisation filter struts 23 are not primarily for providing a securing outward radial force on the vessel wall, for which purpose the stabilisation struts 23 may be generally more flexible than the primary struts 22.

Each strut skeleton 34 is an elongate element of substantially non-biodegradable material, of the type mentioned above, and has a first end 27 and a second end 31. In practice, the strut skeleton extends from the filter hub 29 to the second end 31, the second end 31 in this embodiment being coincident with the second end 30 of the respective fixation filter strut.

In this embodiment, the first end 28 of the fixation filter strut 22 is also the first end 27 of the strut skeleton 34. In other words, the first ends 27 of the strut skeletons 34 are connected to the filter hub 29.

The strut skeleton 34 in this embodiment is a single wire, although may have other forms as described below. The strut skeleton 34 can be thinner and/or softer and/or less rigid than many prior art filter struts.

The strut skeleton 34 has a springiness to provide the fixation filter strut 22 with spring characteristics, and to bias the fixation filter strut 22 towards its radially expanded configuration.

As described above, the material of the strut skeleton 34 may be metal, preferably spring steel, an alloy such as a shape memory material, nickel titanium, cobalt chromium or any other suitable material

The strut skeleton 34 has an operative length, which in this embodiment extends from the filter hub 29 to the second end 31 of the strut skeleton 34. The operative length can be described as a flexure length of the strut skeleton, representative of that part of the strut skeleton 34 designed to flex in order to provide a radially expansive force. The flexure length of the strut skeleton 34 can be a free length of the strut skeleton 34 beyond a coupling region to other parts of the filter 20, in particular the hub 29.

In this embodiment, the operative length is the entire length of the strut skeleton 34.

The strut skeleton 34 has a flexural modulus referred to herein as the first flexural modulus. It is to be understood that where flexural modulus is discussed herein with reference to elongate elements it does not necessarily refer to a single constant value but refers to a profile of values along a length. Accordingly, the term "first flexural modulus" could equally be described as a first flexural modulus profile. Flexural modulus is sometimes called flexural elastic modulus and is the tendency for the strut skeleton, in this case, to bend under an applied force. This is dependent on the geometry of the sample and the degree of deflection due to the force applied during bending. The flexural modulus is related to the area moment of inertia and the degree of deflection under a constant force.

Furthermore, although flexural modulus is discussed herein, the teachings could be applied to the rigidity or bending moment instead or in addition.

The first flexural modulus is configured or selected so that along the operative length of the strut skeleton 34, that is to say along the full operative extent of the strut skeleton 34, the first flexural modulus is uniform or varies in a continuous, that is non-stepped, preferably smooth manner.

The strut skeleton 34 is preferably also configured to have an area moment of inertia, referred to herein as the first area moment of inertia, which is uniform or continuously varying, preferably smooth, along its operative length, that is to say along the full extent of its operative length. The area moment of inertia is calculated with respect to the centre of the strut skeleton 34.

In some embodiments, the strut skeleton varies in at least one of amount and composition along its operative length. Advantageously, such variation in amount and/or composition is gradual along the operative length of the strut skeleton 34 such that the variation in the first flexural modulus and/or first area moment of inertia is continuous, that is without step changes, or preferably smooth, that is also without any sharp changes.

The uniformity or continuous, preferably smooth, variation of the first flexural modulus, and preferably also of the first area moment of inertia, is able to provide strut skeletons 34 having a profile which provides a continuous or preferably smooth curve of radial outward force along the operative lengths of the strut skeletons 34. This can avoid step-changes in the force radially outward produced by the strut skeleton when compressed, and can avoid potential trauma or damage that such a step-change might otherwise produce. Trauma can also result in increased tissue ingrowth. It can also mean that the filter 20 can conveniently be used with different sized vessels as the radial outward force on the vessel is easy to predict, irrespective of where along the strut skeleton 34 it will contact the vessel once the reinforcing element 36 has degraded. It will be appreciated that the point of contact to a vessel wall will vary in dependence upon the relative dimensions of the filter 20 and the vessel, in such a way that in a smaller vessel the filter struts 22, 23 will be compressed more to the point that the struts 22 at least will contact the vessel further along their extent from their free ends.

It is preferable that the first flexural modulus and also the first area moment of inertia is/are a continuous function of distance along the strut skeleton 34. In other words there are no apices, that is to say no step-changes or jumps, in the first flexural modulus, nor preferably in the first area moment of inertia, along the operative length of the strut skeleton 34. This can provide a constant or continuously changing flexural modulus, and preferably also area moment of inertia, along the operative length of the strut skeleton 34. In some embodiments there are no troughs or peaks in the first flexural modulus, nor preferably in the first area moment of inertia, along the operative length of the strut skeleton 34.

In some embodiments, the flexural modulus, strength and/or area moment of inertia of the operative length can be lowest at a second end of the operative length, which is towards the second end 26 of the filter, and greatest at a first end of the operative length, which is towards the first end 24 of the filter 20. This can provide for a small device. In other embodiments, the first flexural modulus and/or the first area moment of inertia is/are substantially constant along the operative length of the strut skeleton 34 as this can make the device easier and cheaper to produce.

In some embodiments, the first flexural modulus and/or the first area moment of inertia along the operative length of the strut skeleton 34 can be a linear function of distance along the strut skeleton 34.

Referring again to Figure 3, the reinforcement element 36 is made of biodegradable material and is provided to reinforce the strut skeleton 34 thereby to increase the flexural modulus and the area moment of inertia of the fixation filter strut 22. Specifically, the reinforcement element 36 causes an increase in the flexural modulus and the area moment of inertia of the resulting fixation filter strut 22 assembly, advantageously sufficient to cause a large enough radial force to be applied to hold the filter 20 within the vessel immediately after implantation, before the fixation filter struts 22 have been encapsulated by tissue by endothelialisation.

In this embodiment, the reinforcement element 36 reinforces the entire operative length of the strut skeleton 34, although this is not essential in every embodiment. The element 36 is attached to the skeleton 34, either by natural adhesion such as by van der Wall's forces, or by adhesive or other bonding. In some embodiments, the strut skeleton and reinforcement element may be provided by a composite material in which both the strut skeleton and reinforcement element are integral.

The reinforcement element 36 may include a biodegradable polymer. In some embodiments, the reinforcement element 36 can be formed of one or more different materials and can include one or more layers or sections along its length.

As used herein, the term "biodegradable" is intended to encompass the terms "bioresorbable", "bioabsorbable", and "bioerodable". Any portion of a medical device of the present invention that is described herein as "biodegradable", "bioresorbable", or "bioerodable" will, over time, lose bulk mass by being degraded, resorbed or eroded by normal biological processes in the body. The prefix "bio" indicates that the erosion occurs under physiological conditions, as opposed to other erosion processes, caused, for example, by high temperature, strong acids or bases, UV light or weather conditions. A biodegradable material has the ability naturally to disappear, for example by dissolving or being broken down into pieces absorbed or secreted by the body, over time *in vivo* in accordance with any biological or physiological mechanism, such as, for example, erosion, degradation, dissolution, chemical depolymerisation including at least acid- and base-catalysed hydrolysis and free radical-induced depolymerisation, enzymatic depolymerisation, absorption and/or resorption within the body. Typically, the material is metabolised or broken down by normal biological processes into metabolites or breakdown products that are substantially non-toxic to the body and are capable of being resorbed and/or eliminated through normal excretory and metabolic processes of the body. As such, biodegradable devices do not require surgical removal.

The reinforcement element 36 may be provided as a coating to the strut skeleton 34, for example by being attached or by being integrally formed, so as to surround the strut skeleton 34 concentrically for example as shown in Figure 4. However, it is also possible for the reinforcement element 36 to be provided only on a part of the surface of the strut skeleton 34, and it is possible for the reinforcement element 36 to be provided at least partially inside, or even completely inside the strut skeleton 34. Examples of configurations of reinforcement elements with respect to the strut skeleton are described below.

The reinforcement element 36 provides the fixation filter strut 22 with a second flexural modulus which is greater than the first flexural modulus. The reinforcement element 36 could be considered to stiffen the strut skeleton 34 in its intended shape.

The fixation filter strut 22 is therefore a composite of the skeleton 34 and reinforcement element 36.

In some embodiments, the reinforcement element 36 is provided in different amounts and/or compositions along the length of the skeleton 34, for example different thicknesses and/or different materials or ratios of materials. However, in embodiments in which the amount or composition of the strut skeleton varies, it is possible to provide a uniform reinforcement element. In either case, the reinforcement element is provided so that the second flexural modulus varies along the operative length of the strut 22. Advantageously, the variation in amount and/or composition of the reinforcement element 36 is gradual along the operative length of the strut 22 such that the variation in the second flexural modulus is continuous, that is without step changes, or preferably smooth, that is also without any sharp changes in gradient.

In preferred embodiments, the second flexural modulus, that is the flexural modulus of the reinforced fixation filter strut is uniform or continuously or smoothly varying along its operative length.

The reinforcement element 36 is preferably of a nature that the fixation filter strut 22 is provided with a second area moment of inertia along its operative length which is uniform or, preferably, varies in a continuous, that is non-stepped, or smooth manner.

For this purpose, the area moment of inertia, or the thickness and therefore the cross-sectional area, of the reinforcement element 36 can vary along the operative length, that is to say can be different at different points along the operative length 32.

As mentioned above, the uniformity or continuous or smooth variation of the second flexural modulus, and preferably also of the second area moment of inertia, is able to provide fixation filter struts having a profile which has a continuous or smooth curve of radial outward force along the operative lengths of the struts when radially compressed. This can avoid step-changes in the force on the filter strut and avoid potential trauma or increased ingrowth that such a step-change might otherwise produce. It can also mean that the filter can conveniently be deployed in different sized vessels as the radial outward force on the vessel is easy to predict irrespective of where along the fixation filter strut it contacts the vessel.

In some embodiments, the second flexural modulus, and preferably also the second area moment of inertia, is/are a continuous function of distance along the strut. In other words there are no apices, that is to say no step changes or jumps, in the second flexural modulus, nor preferably in the second area moment of inertia, along the operative length 32 of the fixation filter strut 22. This can provide a continuously changing flexural modulus, and preferably also area moment of inertia, along the operative length 32 of the fixation filter strut 22. In some embodiments there are no troughs or peaks in the second flexural modulus, nor preferably in the second area moment of inertia, along the operative length 32 of the fixation filter strut 22.

In some embodiments, the flexural modulus, strength and/or area moment of inertia of the operative length 32 is/are lowest at the second end of the operative length 32, which is towards the second end 26 of the filter, and greatest at a first end of the operative length, which is towards the first end 24 of the filter 20.

In some embodiments, the second flexural modulus and/or the second area moment of inertia along the operative length 32 of the fixation filter strut 22 can be a linear function of distance along the strut.

Some existing filters are known in which a small section of biodegradable material can be provided to reinforce particular sections of strut, for example a hinge or flexible point of a filter strut. However, this may in some circumstances provide, at one or more points in the life of the filter, a discontinuity in the flexural modulus and area moment of inertia of the filter strut. This may potentially cause a large amount of the radial force in the filter strut to be focused at or near this point. This might have the potential to be traumatic or cause increased ingrowth of the filter strut. As a result, it is preferred that the skeleton 34 is devoid of any hinge points or similar flexural discontinuities. By providing a smooth profile of flexural modulus, and preferably also of area moment of inertia, both before and after degradation, the risk of this occurring is minimised.

In embodiments of the invention, the reinforcement element 36 is configured so that throughout degradation thereof, the flexural modulus, and preferably also the area moment of inertia, of the fixation filter strut 22 is/are uniform or continuously or smoothly varying along the operative length of the strut 22. Continuous variation can avoid step changes, and smooth variation can avoid sharp changes in gradient. Preferably, throughout degradation of the reinforcement element, the flexural modulus, strength and/or area moment of inertia of the operative length 32 is/are lowest at the second end of the operative length 32 and greatest at the first end of the operative length 32.

In some embodiments, throughout degradation of the reinforcement element 36, the flexural modulus and/or area moment of inertia of the fixation filter strut 22 can be a linear function of distance along the strut.

The fixation filter strut 22 can be considered to be a composite in which the reinforcement element 36 is the matrix and the strut skeleton 34 is the fibre material. Using this composite, the radial force acting on the vessel can be designed within a frame of properties including radial force at implantation, radial force during indwelling, period of reduction for radial force, and final implant radial force.

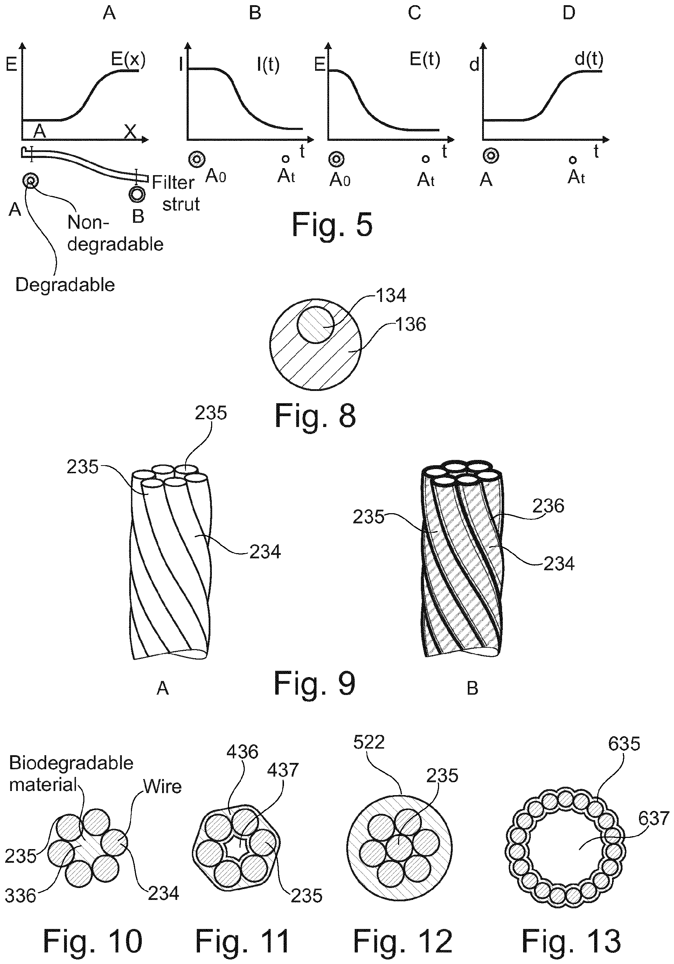

As can be seen in Figure 5, the second flexural modulus for the fixation filter strut 22, that is of the composite structure, varies in a continuous and smooth manner along the strut for the extent of the operative length 32 of the fixation filter strut 22. Figure 5a shows a graph of the flexural modulus against distance along the operative length 32 of the fixation filter strut 22 before implantation. As can be seen in Figure 5a, the flexural modulus varies in a continuous and smooth manner along the operative length 32 of the fixation filter strut 22.

As can be seen in Figure 5a, the flexural modulus of the operative length 32 is lowest at the second end of the operative length 32, which is towards the second end 26 of the filter, and greatest at a first end of the operative length, which is towards the first end 24 of the filter 20.

Figure 5c is a graph of the flexural modulus of a particular cross-section of the fixation filter strut 22 against time. As can be seen from Figure 5c, the flexural modulus of the cross section of the fixation filter strut 22 in question is a continuous function of time. The flexural modulus decreases in a continuous manner until the flexural modulus is simply the flexural modulus of the strut skeleton 34. At all points along the operative length 32 of the fixation filter strut 22, the flexural modulus of the fixation filter strut 22 is a continuous function of time, that is to say that the flexural modulus at each point is constant or varies continuously over time.

Figure 5b is a graph of the area moment of inertia of a particular cross-section of the fixation filter strut 22 against time. As can be seen from Figure 5b, the area moment of inertia of the cross section of the fixation filter strut 22 in question is a continuous function of time. As the reinforcing element degrades, the cross-sectional area decreases. The area moment of inertia decreases in a continuous manner until the area moment of inertia is simply the area moment of inertia of the strut skeleton 34. At all points along the operative length 32 of the fixation filter strut 22, the area moment of inertia of the fixation filter strut 22 is a continuous function of time, that is to say that the area moment of inertia at each point is constant or varies continuously over time.

Figure 5d is a graph of the deflection that a particular cross-section of the fixation filter strut 22 would have, against time, on the basis of an unchanging applied force. When either or both of the flexural modulus and area moment of inertia decrease, the degree of deflection will increase. As can be seen from Figure 5d, the deflection of the cross-section of the fixation filter strut 22 in question would be a continuous function of time. The deflection would increase in a continuous manner until the deflection would be simply the deflection that the strut skeleton 34 would have. At all points along the operative length 32 of the fixation filter strut 22, the deflection that the fixation filter strut 22 would have is a continuous function of time, that is to say that the deflection at each point would be constant or vary continuously over time.

In order to form a filter 20 as described above, a determination is made as to the desired radial force to be exerted by the fixation filter struts 22 on a vessel wall immediately upon implantation of the filter 20, and/or a desired deflection that the fixation filter strut should have on the basis of a predetermined force. This can be considered to be an initial or first force or deflection profile. The first force or deflection profile is uniform or continuously or smoothly varying along the operative length of the fixation filter strut 22.

In addition, a desired final or second radial force is determined for the fixation filter struts 22, which is the radial force that the strut skeletons 23 will exert after the filter 20 has been implanted for a long period of time, once the reinforcing elements 36 have degraded, and/or a desired deflection is determined that the strut skeletons should have on the basis of a predetermined force. This can be considered to be a final or second force or deflection profile. The second force or deflection profile is advantageously uniform or continuously or smoothly varying along the operative length of the strut skeleton 23. The second radial force is less than the first radial force and/or the second deflection is greater than the first deflection.

In addition, it is preferably determined how it is desired that the radial force and/or deflection should change during degradation of the reinforcing element by way of an intermediate force or deflection profile. The intermediate force or deflection profile is uniform or continuously or smoothly varying along the operative length of the fixation filter strut 22 throughout degradation of the reinforcing element.

For each fixation filter strut 22, a strut skeleton 34 can be formed or selected which has the first flexural modulus, the first flexural modulus being uniform or continuously or smoothly varying along the operative length of the strut skeleton 34, and preferably also the first area moment of inertia which is uniform or continuously or smoothly varying along the operative length of the strut skeleton 34, so that the strut skeleton provides the desired second force and/or deflection profile.

The strut skeleton may for example be a metal wire of a fixed size. The strut skeleton can also have a desired transversal section, for example the strut skeleton can have a height:width ratio 6:1.

The strut skeleton 34 is reinforced with the reinforcing element 36 in accordance with a reinforcement profile in order to provide the fixation filter strut with the second flexural modulus, preferably the second area moment of inertia, and preferably a degradation profile, so that the fixation filter strut 22 provides the desired force and/or deflection before, during and after degradation of the reinforcement element. The strut skeleton can for example be reinforced by having the reinforcing element 36 attached to it or by being integrally formed with the reinforcing element.

The degradation profile is a desired transition as a function of time from the second flexural modulus to the first flexural modulus, and preferably also from the second area moment of inertia to the first area moment of inertia, so that throughout the degradation, the flexural modulus of the fixation filter strut 22 is uniform or varies continuously or smoothly along the operative length of the fixation filter strut 22 for the extent of the operative length 32 of the fixation filter strut 22, and preferably also so that, throughout the degradation, the area moment of inertia of the fixation filter strut 22 is uniform or varies continuously along the operative length of the fixation filter strut 22 for the extent of the operative length 32 of the fixation filter strut 22.

In general, the degradation profile provides for the flexural modulus, and preferably also the area moment of inertia, to be a continuous function of time throughout the degradation of the reinforcing element 36 at each point along the operative length 32 of the fixation filter strut 22.

The degradation profile preferably provides for the flexural modulus and preferably also the area moment of inertia, to be uniformly reduced over time along the full extent of the operative length of the fixation filter strut 22.

The uniformity or continuous or smooth variation of the flexural modulus, and preferably also of the area moment of inertia, throughout degradation, is able to provide fixation filter struts 22 having a profile which always provides a continuous or smooth curve of radial outward force along the operative lengths of the fixation filter struts 22. This can avoid step-changes in the force on the struts 22 and avoid potential trauma or increased ingrowth that such a step-change might otherwise produce. It can also mean that the filter 20 can conveniently be used with different sized vessels as the radial outward force on the vessel is easy to predict irrespective of where along the strut 22 it will contact the vessel.

By way of example only, the deflection of a fixed beam with a single load at the free end is $\dfrac{FL^3}{3EI}$ where F is the force, L the length, E the flexural modulus (which can be denoted E_{bend}), and I the area moment of inertia. The area moment of inertia of a cylinder is

$$I = \frac{\pi r^4}{4}$$ where r is the radius of the cylinder.

The reinforcement profile can also provide a desired transversal section to the fixation filter strut 22 and/or can provide a desired tranversal flexural modulus, area moment of inertia, and/or rigidity to minimise filter strut entanglement, for example, the fixation filter strut can be designed to have a height:width ratio <1.

The transversal properties can be determined using similar evaluations as described for the longitudinal profiles. For example, the strut skeleton can be formed or selected, and/or the reinforcement profile determined, so as to provide a desired first, second and/or intermediate transversal flexural modulus, area moment of inertia and/or force or deflection, which may be uniform or continuously or smoothly varying through the width or height of the strut, before, during, throughout, and/or after degradation of the reinforcing element.

A first end of each of the fixation filter struts 22 and in this embodiment the first ends of the stabilisation filter struts are then coupled to the filter hub 29.

In order to implant the filter 20, the filter 20 can be biased into a radially compressed configuration and held within a sheath of a delivery device. The delivery device can be advanced endoluminally to a treatment site. At the treatment site, the filter 20 can be deployed, for example by retracting the sheath, thereby to allow the filter struts 22, 23 to expand radially into the deployed configuration shown in Figure 2.

Owing to the increased flexural modulus and area moment of inertia provided by the reinforcement element 36, the fixation filter struts 22 of the filter 20 initially apply a relatively high radial force on the vessel wall so as to secure the filter 20 in position in the vessel. Furthermore, because of the continuous or smooth nature of the flexural modulus and preferably area moment of inertia of the fixation filter struts 22 along the operative length 32 of the fixation filter struts 22, the forces on the fixation filter struts 22 are continuously or smoothly distributed along the fixation filter struts 22, thereby minimising the risk of any disproportionately high stress points on the fixation filter struts 22.

As described above, over time, the reinforcement elements 36 degrade. However, in preferred embodiments, because of the nature of the reinforcing profile by which the fixation filter struts 22 were reinforced, throughout degradation of the reinforcement elements 36, the flexural modulus of the fixation filter struts 22 and preferably also the area moment of inertia of the fixation filter struts is/are uniform or continuously or smoothly varying along the operative length 32 of the fixation filter struts 22, thereby minimising the risk of the development of a region of high stress.

Eventually, after the filter 20 has been implanted for a while, and the fixation filter struts 22 have become encapsulated by tissue, the entirety of the reinforcement elements 36 will have degraded, and the fixation filter struts 22 will have reduced to just the strut skeletons 34. These strut skeletons 34 will have a flexural modulus and preferably also an area moment of inertia which is/are uniform or continuously or smoothly varying along their operative lengths, thereby continuing to avoid points of high stress. However, the radial force exerted by the strut skeletons 34 will be significantly less than the radial force exerted by the fixation filter struts 22 immediately after implantation.

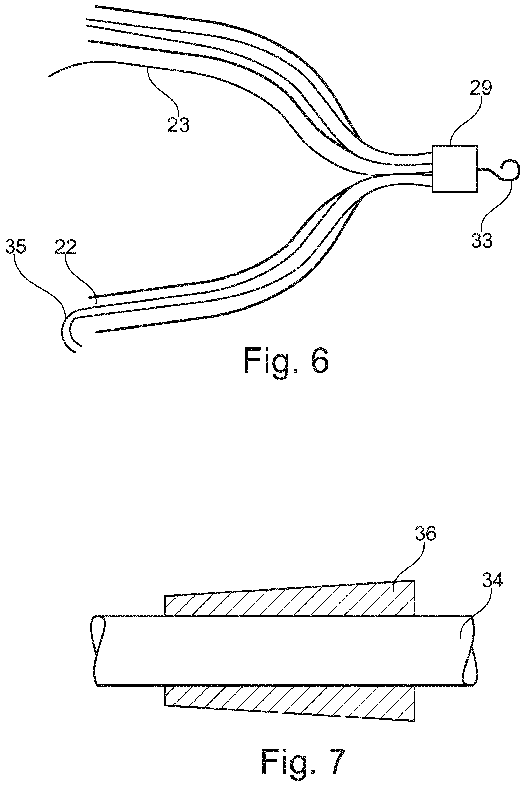

In one particular example, shown in Figures 6 and 7, the strut skeleton 34 of each fixation filter strut is a spring steel wire with a uniform diameter along the operative length. The reinforcement element 36 is a polymer and is attached to the strut skeleton with a varying diameter or cross-section along the operative length so that, upon implantation, the flexural modulus is greatest at the first end of the operative length, that is towards the hub, and decreases along the operative length. It could be said that the reinforcement element 36 is slightly conical in this embodiment.

In another example, the strut skeleton of each fixation filter strut is a spring steel wire with a uniform diameter along the operative length. The reinforcement element is a combination of different polymers and is attached to the strut skeleton with a uniform diameter or cross-section along the operative length but with different polymer materials or different ratios of polymer materials in a polymer material mix along the operative length so that, upon implantation, the flexural modulus is greatest at the first end of the operative length, that is towards the hub, and decreases along the operative length. However, in a modification of this example, the diameter or cross-section of the reinforcement element can be varying.

In another example, the strut skeleton of each fixation filter strut is a spring steel element with a tapering diameter along the operative length providing a flexural modulus which is greatest at the first end of the operative length, that is towards the hub, and which decreases along the operative length. The reinforcement element is a polymer and is attached to the strut skeleton in a uniform manner along the operative length so that, upon implantation, the second flexural modulus is, as a result of the variation in the first flexural modulus, greatest at the first end of the operative length, that is towards the hub, and decreases along the operative length. In this example, the strut skeleton can be provided by first and second strands of rectangular or flat but tapering cross-section, held apart by the reinforcement element. Furthermore, in a modification of this example, the amount and/or composition of the reinforcement element can be varying.

A wide variety of materials is known for the biodegradable reinforcement element, such as metals and polymers which can be for corroding to being fully absorbed. As these are well known in the art a long list is not necessary herein. Some examples, though, include biodegradable metals such as magnesium, zinc, iron, and other metals and metal alloys, and biodegradable polymers for instance formed from polyglycolic or polylactic acid and copolymers thereof. Reference is made to Biodegradable Metals for Cardiovascular Stent Application: Interests and New Opportunities by Maryam Moravej and Diego Mantovani, Int J. Mol Sci 2011; 12(7):4250-4270. Possible options also include polyglycolide (PGA), one of the stiffest bioabsorbable polymers used in medical devices with a E-moduli=7.0GPa and ultimate tensile strength (UTS=70MPa). The strength and stiffness of such a polymer itself would probably last for at least 1-2 months, but the mass would be there for 6-12 months. PGA undergoes bulk hydrolysis, which means that it takes up water from the environment and the molecular chains are cleaved and ultimately the molecular weight diminishes, and thereby the stiffness and strength is reduced. The filter strut would lose its strength and stiffness sometime between 2 and 12 months. A further possible option is poly(L-lactide). It keeps its strength and stiffness up till 6 months, but the mass remains until 24 months. A further possible option is a combination by co-polymerization, to create poly(lactide-co-glycolide). If the strut skeleton is covered by a surface erosive polymer, the point at which strength is lost is more gradual as the layer of polymer slowly is diminished. The polymer does not have to be hydrolytically degradable, it could be enzymatically degradable.

The reinforcement element could be electrolytically degradable, and/or could corrode such as with the corrosion of iron, magnesium metal or metal alloys.

The reinforcement element and strut skeleton may form a composite material where the degradable element is stronger than the basis.

As described above, it is not necessary in all embodiments for the fixation filter struts 22 to be coated evenly with the reinforcement elements 36 so that in the initial condition before implantation, the strut skeleton 34 is located coaxially within the fixation filter strut 22 as shown in Figure 4.

Figures 8 to 13 show other embodiments with different configurations of the strut skeletons with respect to the reinforcement elements. These configurations can be used for one or more or all of the fixation filter struts. The strut skeleton can for example be a braided structure, a coiled structure, or a wire.

In Figure 8, the strut skeleton 134 is located eccentrically, or off-centre, within the reinforcement element 136. This can be advantageous, for example, if in the initial condition, the strut skeleton is positioned radially inwardly with respect to a centre of the fixation filter strut. Accordingly, as the biodegradable material of the reinforcement element 136 degrades, there is a greater distance between the initial position of the vessel wall and the strut skeleton 134 into which the vessel wall can relax. However, in other embodiments, the strut skeleton can be positioned radially outwardly with respect to the centre of the filter strut.

Figure 9a shows a strut skeleton 234 which includes a plurality of wires or strands 235 which are twisted together in a helical fashion. The wires can be made of fibre material with a pitch from 0º to 90º. It is not necessary for the wires to be twisted in a helical fashion in all embodiments. The wires can for example be straight. For example, the strut skeleton can comprise two flat adjacent wires. However, the helical twisting shown in Figure 9a reduces the risk of the wires becoming separated once the reinforcement element has degraded.

Figure 9b shows the strut skeleton 234 of Figure 9a reinforced with a reinforcement element 236. As can be seen in Figure 9b, the wires 235 in this embodiment have biodegradable material of the reinforcing element between and around the wires 235, coating the wires and filling gaps between the wires. This effectively creates a fixation filter strut comprising a plurality of strands or wires which are coupled with biodegradable material.

Although Figure 9b shows the reinforcement element 236 coating the wires as well as between the wires or strands 235, this is not necessary in all embodiments. In some embodiments, the reinforcement element may only be between the wires or strands. For example, for the embodiment with two flat adjacent wires, the reinforcement element may be between the two flat wires and couple them together.

In Figure 10, the biodegradable material 336 is located substantially entirely within the fixation filter strut 234 and within a space surrounded by the wires or strands 235.

In Figure 11, each of the wires or strands 235 of the strut skeleton 234 is coated and substantially surrounded by biodegradable material 436. In this embodiment there is an open zone 437 in the centre of the fixation filter strut. However, in other embodiments, such as that shown in Figure 12, there is no open zone in the centre. In the embodiment of Figure 12, the open zone is filled with an additional central wire or strand 235, so that the entire fixation filter strut 522 in this embodiment is substantially solid before implantation.

Although the embodiments of Figures 9 to 12 show a fixation filter strut comprising 6 wires or strands, in other embodiments a different number of wires or strands such as more or less than 6 can be used. For example, the embodiment of Figure 13 is similar in many respects to the embodiment of Figure 11, except that the embodiment of Figure 13 includes a larger number of wires or strands 635 with the result that the individual wires or strands 635 are of a smaller diameter than the strands or wires 235 of the embodiment of Figure 11 and that there is a larger open zone 637 in the centre of the fixation filter strut.

Although the above embodiments relate to a filter, the method of embodiments of the invention can be used for forming struts of any implantable medical device which can have a second flexural modulus upon implantation reducing to a first flexural modulus after a length of time within a vessel.

Although in above embodiments the strut skeleton is non-degradable, it is not excluded that the strut skeleton can be biodegradable, for example taking longer to biodegrade than the reinforcing element. For example, the struts can include a biodegradable metal coated with a biodegradable polymer. In such a case, as for the reinforcing element, the strut skeleton can be configured to biodegrade such that its flexural modulus and/or its area moment of inertia is always uniform or continuously varying.

**Claims**

1. An implantable medical device (20) including a plurality of struts (22) arranged in a generally conical form; each strut having a first end (28), a second end (30), and an operative length (32) between the first and second ends; each strut (22) being able to flex along the operative length (32) thereof and including:

   a strut skeleton (34) having a first flexural modulus; and
   a reinforcement element (36) extending along the operative length (32) and attached to the strut skeleton, wherein the reinforcement element (36) and the strut skeleton (34) together have a second flexural modulus greater than the first flexural modulus, wherein at least one of the reinforcement element (36) and the strut skeleton (34) varies in at least one of amount and composition continuously along the operative length (32) such that the second flexural modulus varies in a continuous manner along the operative length;
   wherein the reinforcement element (36) is biodegradable thereby changing the flexural modulus of the strut from the second flexural modulus to the first flexural modulus as the reinforcement element biodegrades.

2. An implantable medical device (20) according to claim 1, including a hub element (29) to which the first end (28) of each strut is attached, the struts (22) extending at least partially radially from the hub element (29) towards the second end (30) of the strut; wherein the operative length (32) of each strut optionally extends from the hub element (29) to the second end (30) of the strut (22).

3. An implantable medical device (20) according to any preceding claim, wherein a radial outward force provided by each strut (22) is substantially uniform or continuously varying along the operative length (32).

4. An implantable medical device (20) according to any preceding claim, wherein the first flexural modulus is uniform or varies in a continuous manner along the operative length.

5. An implantable medical device (20) according to any preceding claim, wherein each strut (22) has an area moment of inertia which is one of uniform or varying in a continuous manner along the operative length (32) of the strut (22); optionally wherein each strut (22) has an area moment of inertia which is one of uniform or varying in a continuous manner along the operative length (32) of the strut (22) throughout degradation of the reinforcement element (36).

6. An implantable medical device (20) according to any preceding claim, wherein each strut skeleton (34) has an area moment of inertia which is one of uniform or varying in a continuous manner along the operative length (32).

7. An implantable medical device (20) according to any preceding claim, wherein each reinforcement element (36) is integral with its respective strut skeleton (34).

8. An implantable medical device (20) according to any preceding claim, wherein each reinforcement element (36) has a varying composition along the operative length (32) of the strut (22).

9. An implantable medical device (20) according to any preceding claim, wherein each reinforcement element (36, 136, 236, 436) is in the form of a coating on the strut skeleton (34, 134, 234); and/or

   wherein each strut skeleton (234) is formed of a plurality of wires or strands (235, 635); and/or
   wherein each strut skeleton (234) is tubular and the reinforcement element (336) thereof is at least partially within the strut skeleton (234).

10. A method of forming an implantable medical device (20), including:

    forming a plurality of struts (22), each strut having a first end (28), a second end (30), an operative length (32) between the first and second ends and a strut skeleton (34) having a first flexural modulus;

forming a reinforcement element (36) extending along the operative length (32) of each strut (22), the reinforcement element (36) and the strut skeleton (34) together having a second flexural modulus greater than the first flexural modulus, wherein at least one of the reinforcement element (36) and the strut skeleton (34) varies in at least one of amount and composition continuously along the operative length (32) such that the second flexural modulus varies in a continuous manner along the operative length (32); arranging the struts (22) into a generally conical frame;

wherein the reinforcement element (36) is biodegradable thereby changing the flexural modulus of the strut (22) from the second flexural modulus to the first flexural modulus as the reinforcement element (36) biodegrades.

**11.** A method according to claim 10, wherein forming a reinforcement element (36) extending along the operative length (32) of each strut (22) provides the strut, the strut being a filter strut, with an area moment of inertia which is one of uniform or varying in a continuous manner along the operative length (32) of the strut (22).

**12.** A method according to any of claims 10 to 11, wherein forming each strut (22) includes:

selecting a desired first force or deflection profile for the strut; and

determining a reinforcement profile for the strut skeleton in accordance with the first force or deflection profile and forming the reinforcement element (36) extending along the operative length (32) of each strut (22) in accordance with the reinforcement profile thereby to provide the strut (22) with the second flexural modulus and the first force or deflection profile; wherein the first force or deflection profile is optionally substantially uniform or continuously varying along the operative length of the strut.

**13.** A method according to claim 12, wherein the reinforcement profile of the reinforcement element (36) for each strut skeleton (34) provides the strut (22) with a flexural modulus which is one of uniform or varying continuously along the operative length (32) of the strut throughout degradation of the reinforcing element (36).

**14.** A method according to any one of claims 10 to 13, including the step of selecting a desired second force or deflection profile for each strut skeleton (34); and forming or selecting the strut skeleton (34) in accordance with the second force or deflection profile such that the strut skeleton (34) has the first flexural modulus and the second force or deflection profile.

**Patentansprüche**

**1.** Implantierbare medizinische Vorrichtung (20), aufweisend eine Vielzahl von Streben (22), die in einer allgemein konischen Form angeordnet sind, wobei jede Strebe ein erstes Ende (28), ein zweites Ende (30) und eine Betriebslänge (32) zwischen dem ersten und dem zweiten Ende hat, wobei sich jede Strebe (22) entlang der Betriebslänge (32) davon biegen kann und Folgendes aufweist:

ein Strebenskelett (34) mit einem ersten Biegemodul und

ein Verstärkungselement (36), das sich entlang der Betriebslänge (32) erstreckt und an dem Strebenskelett angebracht ist, wobei das Verstärkungselement (36) und das Strebenskelett (34) zusammen einen zweiten Biegemodul haben, der größer als der erste Biegemodul ist, wobei das Verstärkungselement (36) und/oder das Strebenskelett (34) hinsichtlich der Anzahl und/oder der Zusammensetzung kontinuierlich entlang der Betriebslänge (32) variieren, so dass der zweite Biegemodul kontinuierlich entlang der Betriebslänge variiert, wobei das Verstärkungselement (36) biologisch abbaubar ist, wodurch sich der Biegemodul der Strebe von dem zweiten Biegemodul zu dem ersten Biegemodul ändert, während das Verstärkungselement biologisch abgebaut wird.

**2.** Implantierbare medizinische Vorrichtung (20) nach Anspruch 1, aufweisend ein Ansatzelement (29), an dem das erste Ende (28) jeder Strebe angebracht ist, wobei sich die Streben (22) mindestens teilweise radial von dem Ansatzelement (29) zu dem zweiten Ende (30) der Strebe erstrecken, wobei sich die Betriebslänge (32) jeder Strebe optional von dem Ansatzelement (29) zu dem zweiten Ende (30) der Strebe (22) erstreckt.

**3.** Implantierbare medizinische Vorrichtung (20) nach einem der vorhergehenden Ansprüche, wobei eine von jeder Strebe (22) bereitgestellte nach außen gehende Kraft entlang der Betriebslänge (32) im Wesentlichen einheitlich ist oder kontinuierlich variiert.

**4.** Implantierbare medizinische Vorrichtung (20) nach einem der vorhergehenden Ansprüche, wobei der erste Biegemodul entlang der Betriebslänge einheitlich ist oder kontinuierlich variiert.

**5.** Implantierbare medizinische Vorrichtung (20) nach einem der vorhergehenden Ansprüche, wobei jede Strebe (22) ein Flächenträgheitsmoment hat, das entlang der Betriebslänge (32) der Strebe (22) einheitlich ist oder kontinuierlich variiert, optional wobei jede Strebe (22) ein Flächenträgheitsmoment hat, das während des Abbaus des Verstärkungselements (36) entlang der Betriebslänge (32) der Strebe (22) einheitlich ist oder kontinuierlich variiert.

6. Implantierbare medizinische Vorrichtung (20) nach einem der vorhergehenden Ansprüche, wobei jedes Strebenskelett (34) ein Flächenträgheitsmoment hat, das entlang der Betriebslänge (32) einheitlich ist oder kontinuierlich variiert.

7. Implantierbare medizinische Vorrichtung (20) nach einem der vorhergehenden Ansprüche, wobei jedes Verstärkungselement (36) mit seinem jeweiligen Strebenskelett (34) integral ist.

8. Implantierbare medizinische Vorrichtung (20) nach einem der vorhergehenden Ansprüche, wobei jedes Verstärkungselement (36) eine variierende Zusammensetzung entlang der Betriebslänge (32) der Strebe (22) hat.

9. Implantierbare medizinische Vorrichtung (20) nach einem der vorhergehenden Ansprüche, wobei jedes Verstärkungselement (36, 136, 236, 436) in der Form einer Beschichtung auf dem Strebenskelett (34, 134, 234) vorliegt und/oder

wobei jedes Strebenskelett (234) aus einer Vielzahl von Drähten oder Strängen (235, 635) gebildet ist und/oder
wobei jedes Strebenskelett (234) rohrförmig ist und das Verstärkungselement (336) davon mindestens teilweise in dem Strebenskelett (234) ist.

10. Verfahren zum Bilden einer implantierbaren medizinischen Vorrichtung (20), aufweisend:

Bilden einer Vielzahl von Streben (22), wobei jede Strebe ein erstes Ende (28), ein zweites Ende (30), eine Betriebslänge (32) zwischen dem ersten und dem zweiten Ende und ein Strebenskelett (34) mit einem ersten Biegemodul hat,
Bilden eines Verstärkungselements (36), das sich entlang der Betriebslänge (32) jeder Strebe (22) erstreckt, wobei das Verstärkungselement (36) und das Strebenskelett (34) zusammen einen zweiten Biegemodul haben, der größer als der erste Biegemodul ist, wobei das Verstärkungselement (36) und/oder das Strebenskelett (34) hinsichtlich der Anzahl und/oder der Zusammensetzung kontinuierlich entlang der Betriebslänge (32) variieren, so dass der zweite Biegemodul kontinuierlich entlang der Betriebslänge (32) variiert,
Anordnen der Streben (22) zu einem allgemein konischen Rahmen,
wobei das Verstärkungselement (36) biologisch abbaubar ist, wodurch sich der Biegemodul der Strebe (22) von dem zweiten Biegemodul zu dem ersten Biegemodul ändert, während das Verstärkungselement (36) biologisch abgebaut wird.

11. Verfahren nach Anspruch 10, wobei die Strebe, bei der es sich um eine Filterstrebe handelt, durch Bilden eines Verstärkungselements (36), das sich entlang der Betriebslänge (32) jeder Strebe (22) erstreckt, mit einem Flächenträgheitsmoment versehen wird, das entlang der Betriebslänge (32) der Strebe (22) einheitlich ist oder kontinuierlich variiert.

12. Verfahren nach einem der Ansprüche 10 bis 11, wobei das Bilden jeder Strebe (22) Folgendes aufweist:

Wählen eines gewünschten ersten Kraft- oder Auslenkungsprofils für die Strebe und
Bestimmen eines Verstärkungsprofils für das Strebenskelett gemäß dem ersten Kraft- oder Auslenkungsprofils und Bilden des sich entlang der Betriebslänge (32) jeder Strebe (22) erstreckenden Verstärkungselements (36) gemäß dem Verstärkungsprofil, wodurch die Strebe (22) mit dem zweiten Biegemodul und dem ersten Kraft- oder Auslenkungsprofils versehen wird, wobei das erste Kraft- oder Auslenkungsprofils entlang der Betriebslänge der Strebe im Wesentlichen einheitlich ist oder kontinuierlich variiert.

13. Verfahren nach Anspruch 12, wobei das Verstärkungsprofil des Verstärkungselements (36) für jedes Strebenskelett (34) einen Biegemodul für die Strebe (22) bereitstellt, der während des Abbaus des Verstärkungselements (36) entlang der Betriebslänge (32) der Strebe einheitlich ist oder kontinuierlich variiert.

14. Verfahren nach einem der Ansprüche 10 bis 13, aufweisend den Schritt des Wählens eines gewünschten zweiten Kraft- oder Auslenkungsprofils für jedes Strebenskelett (34) und
Bilden oder Wählen des Strebenskeletts (34) gemäß dem zweiten Kraft- oder Auslenkungsprofil, so dass das Strebenskelett (34) den ersten Biegemodul und das zweite Kraft- oder Auslenkungsprofil hat.

**Revendications**

1. Dispositif médical implantable (20) comportant une pluralité d'entretoises (22) disposées selon une forme généralement conique ; chaque entretoise ayant une première extrémité (28), une deuxième extrémité (30) et une longueur fonctionnelle (32) entre les première et deuxième extrémités ; chaque entretoise (22) pouvant fléchir le long de sa longueur fonctionnelle (32) et comportant :

   un squelette d'entretoise (34) ayant un premier module d'élasticité en flexion ; et
   un élément de renforcement (36) s'étendant le long de la longueur fonctionnelle (32) et fixé au squelette d'entretoise, l'élément de renforcement (36) et le squelette d'entretoise (34) ayant ensemble un deuxième module d'élasticité en flexion supérieur au premier module d'élasticité en flexion, au moins l'un parmi l'élément de renforcement (36) et le squelette d'entretoise (34) variant en quantité et/ou en composition en continu le long de la longueur fonctionnelle (32), de sorte que le deuxième module d'élasticité en flexion varie de manière continue le long de la longueur fonctionnelle ;
   l'élément de renforcement (36) étant biodégradable, modifiant ainsi le module d'élasticité en flexion de l'entretoise du deuxième module d'élasticité en flexion au premier module d'élasticité en flexion à mesure que l'élément de renforcement se décompose.

2. Dispositif médical implantable (20) selon la revendication 1, comportant un élément formant moyeu (29) auquel la première extrémité (28) de chaque entretoise est fixée, les entretoises (22) s'étendant au moins partiellement radialement à partir de l'élément formant moyeu (29) vers la deuxième extrémité (30) de l'entretoise ;
   la longueur fonctionnelle (32) de chaque entretoise s'étendant éventuellement de l'élément formant moyeu (29) à la deuxième extrémité (30) de l'entretoise (22).

3. Dispositif médical implantable (20) selon l'une quelconque des revendications précédentes, une force vers l'extérieur radiale fournie par chaque entretoise (22) étant sensiblement uniforme ou variant en continu le long de la longueur fonctionnelle (32).

4. Dispositif médical implantable (20) selon l'une quelconque des revendications précédentes, le premier module d'élasticité en flexion étant uniforme ou variant de manière continue le long de la longueur fonctionnelle.

5. Dispositif médical implantable (20) selon l'une quelconque des revendications précédentes, chaque entretoise (22) ayant un moment d'inertie de surface qui est uniforme ou varie de manière continue le long de la longueur fonctionnelle (32) de l'entretoise (22) ; éventuellement chaque entretoise (22) ayant un moment d'inertie de surface qui est uniforme ou varie de manière continue le long de la longueur fonctionnelle (32) de l'entretoise (22) tout au long de la décomposition de l'élément de renforcement (36).

6. Dispositif médical implantable (20) selon l'une quelconque des revendications précédentes, chaque squelette d'entretoise (34) ayant un moment d'inertie de surface qui est uniforme ou varie de manière continue le long de la longueur fonctionnelle (32).

7. Dispositif médical implantable (20) selon l'une quelconque des revendications précédentes, chaque élément de renforcement (36) faisant partie intégrante de son squelette d'entretoise (34) respectif.

8. Dispositif médical implantable (20) selon l'une quelconque des revendications précédentes, chaque élément de renforcement (36) ayant une composition variant le long de la longueur fonctionnelle (32) de l'entretoise (22).

9. Dispositif médical implantable (20) selon l'une quelconque des revendications précédentes, chaque élément de renforcement (36, 136, 236, 436) étant sous la forme d'un revêtement sur le squelette d'entretoise (34, 134, 234) ; et/ou

   chaque squelette d'entretoise (234) étant formé d'une pluralité de fils ou filaments (235, 635) ; et/ou
   chaque squelette d'entretoise (234) étant tubulaire et son élément de renforcement (336) étant au moins partiellement à l'intérieur du squelette d'entretoise (234).

10. Procédé de formation d'un dispositif médical implantable (20), comportant :

   la formation d'une pluralité d'entretoises (22), chaque entretoise ayant une première extrémité (28), une deuxième extrémité (30), une longueur fonctionnelle (32) entre les première et deuxième extrémités et un squelette d'entretoise (34) ayant un premier module d'élasticité en flexion ;
   la formation d'un élément de renforcement (36) s'étendant le long de la longueur fonctionnelle (32) de chaque entretoise (22), l'élément de renforcement (36) et le squelette d'entretoise (34) ayant ensemble un deuxième module d'élasticité en flexion supérieur au premier module d'élasticité en flexion, au moins l'un parmi l'élément de renforcement (36) et le squelette d'entretoise (34) variant en quantité et/ou en composition en continu le long de la longueur fonctionnelle (32), de sorte que le deuxième module d'élasticité en flexion varie de manière continue le long de la longueur fonctionnelle (32) ;
l'agencement des entretoises (22) selon une forme généralement conique ;
l'élément de renforcement (36) étant biodégradable, modifiant ainsi le module d'élasticité en flexion de l'entretoise (22) du deuxième module d'élasticité en flexion au premier module d'élasticité en flexion à mesure que l'élément de renforcement (36) se décompose.

11. Procédé selon la revendication 10, la formation d'un élément de renforcement (36) s'étendant le long de la longueur fonctionnelle (32) de chaque entretoise (22) fournissant à l'entretoise, l'entretoise étant une entretoise de filtre, un moment d'inertie de surface qui est uniforme ou varie de manière continue le long de la longueur fonctionnelle (32) de l'entretoise (22).

12. Procédé selon l'une quelconque des revendications 10 et 11, la formation de chaque entretoise (22) comportant :

la sélection d'un premier profil de force ou de flexion souhaité pour l'entretoise ; et
la détermination d'un profil de renforcement pour le squelette d'entretoise selon le premier profil de force ou de flexion et la formation de l'élément de renforcement (36) s'étendant le long de la longueur fonctionnelle (32) de chaque entretoise (22) selon le profil de renforcement pour fournir ainsi à l'entretoise (22) le deuxième module d'élasticité en flexion et le premier profil de force ou de flexion ; le premier profil de force ou de flexion étant éventuellement sensiblement uniforme ou variant en continu le long de la longueur fonctionnelle de l'entretoise.

13. Procédé selon la revendication 12, le profil de renforcement de l'élément de renforcement (36) pour chaque squelette d'entretoise (34) fournissant à l'entretoise (22) un module d'élasticité en flexion qui est uniforme ou varie en continu le long de la longueur fonctionnelle (32) de l'entretoise tout au long de la décomposition de l'élément de renforcement (36).

14. Procédé selon l'une quelconque des revendications 10 à 13, comportant l'étape de sélection d'un deuxième profil de force ou de flexion souhaité pour chaque squelette d'entretoise (34) ; et
la formation ou la sélection du squelette d'entretoise (34) selon le deuxième profil de force ou de flexion de sorte que le squelette d'entretoise (34) a le premier module d'élasticité en flexion et le deuxième profil de force ou de flexion.

Fig. 1

Fig. 2

Wire

Fig. 3

Spring wire

Biodegradable material

Fig. 4

Fig. 6

Fig. 7

Fig. 5

Fig. 8

Fig. 9

Fig. 10   Fig. 11   Fig. 12   Fig. 13

## REFERENCES CITED IN THE DESCRIPTION

**Patent documents cited in the description**

- US 8092484 B
- US 20120143238 A
- US 20030153972 A
- EP 2208479 A
- US 20030135265 A
- US 20080281393 A
- US 20120130470 A
- US 20120221040 A
- US 20130006294 A
- US 20130204349 A
- US 6517559 B
- US 7575584 B
- US 8162970 B
- WO 2013106746 A
- WO 2009041664 A
- US 20100016940 A
- GB 2531587 A
- WO 2015192019 A

**Non-patent literature cited in the description**

- Celect Interior Vena Cava Wall Strut Perforation Begets Additional Strut Perforation. *Journal of Vascular and Interventional Radiology,* October 2015, vol. 26 (10), 1510-1518
- **MARYAM MORAVEJ ; DIEGO MANTOVANI.** Biodegradable Metals for Cardiovascular Stent Application: Interests and New Opportunities. *Int J. Mol Sci,* 2011, vol. 12 (7), 4250-4270